# NOUVEAU FASCICULE DE BREVET EUROPEEN

(11) **EP 0 742 834 B2**
(45) Date de publication et mention de la décision concernant l'opposition: **03.08.2005**
(45) Mention de la délivrance du brevet: 20.09.2000
(21) Numéro de dépôt: 95941755.1
(22) Date de dépôt: 01.12.1995
(51) Int. Cl.: C12N 15/86, C12N 15/10, C12N 7/00, C12N 7/01

(54) **PROCEDE DE PREPARATION D'UN VECTEUR VIRAL D'AU MOINS 20KB PAR RECOMBINAISON HOMOLOGUE INTERMOLECULAIRE DANS UNE CELLULE PROCARYOTE**
VERFAHREN ZUR HERSTELLUNG VON VIRALEN VEKTOREN VON MINDESTENS 20KB DURCH INTERMOLEKULARE HOMOLOGE REKOMBINATION IN EINER PROKARYOTISCHEN ZELLE
METHOD OF PREPARING A VIRAL VECTOR OF AT LEAST 20KB BY INTERMOLECULAR RECOMBINATION IN A PROCARYOTIC CELL

(30) Priorité: 01.12.1994 FR 9414470
(43) Date de publication de la demande: 20.11.1996
(73) Titulaire: TRANSGENE S.A., 67082 Strasbourg Cédex (FR)
(72) Inventeur: CHARTIER, Cécile, F-67000 Strasbourg (FR); DEGRYSE, Eric, F-67100 Strasbourg (FR)
(74) Mandataire: Warcoin, Jacques
(86) Numéro de dépôt international: PCT/FR1995/001590
(87) Numéro de publication internationale: WO 1996/017070

(56) Documents cités:
- WO-A-95/03400
- PROCEEDINGS OF THE NATIONAL ACADEMY OF SCIENCES OF USA, vol. 91, no. 13, 1994 WASHINGTON US, pages 6186-6190, KETNER, G. ET AL. 'Efficient manipulation of the human adenovirus genome as an efficious yeast artificial chromosome clone'
- JOURNAL OF BIOTECHNOLOGY., vol. 39, no. 2, 1995 AMSTERDAM NL, DEGRYSE, E. 'Evaluation of Escherichia coli recBC sbcBC mutants for cloning by recombination in vivo'
- VIRUS RESEARCH, vol. 28, no. 2, 1993 pages 153-170, SCHORR, J. & DOERFLER 'Non-homologous recombination between adenovirus and AcNPV DNA fragments in cell-free extracts from insect Spodoptera frugiperda nuclei'
- SCIENCE, vol. 196, no. 4286, 1977 LANCASTER, PA US, PERRICAUDET, M. ET AL. 'Excision and recombination of adenovirus DNA fragments in Escherichia coli'
- NUCLEIC ACIDS RESEARCH, vol. 21, no. 4, 1993 pages 817-821, BOYD, A.C. 'Turbo cloning: a fast, efficient method for cloning PCR products and other blunt-ended DNA fragments into plasmids'
- YEAST, vol. 11, no. 7, 1995 pages 629-640, DEGRYSE, E. ET AL. 'In vivo cloning by homologous recombination in yeast using a two-plasmid-based system'
- JOURNAL OF MOLECULAR BIOLOGY, vol. 227, no. 1, 1992 pages 72-80, LUISI-DE LUCA, C. & KOLODNER, R.D. 'Effects of terminal non-homology on intramolecular recombination of linear plasmid substrates in Escherichia coli'
- PROCEEDINGS OF THE NATIONAL ACADEMY OF SCIENCES OF USA., vol. 90, no. 15, Septembre 1993 WASHINGTON US, pages 7356-7360, TATZELT, J. ET AL. 'Fractionated nuclear extracts from hamster cells catalyze cell-free recombination at selective sequences between adenovirus DNA and a hamster preinsertion site'
- GENE THERAPY, vol. 2, no. 4, Juin 1995 pages 263-268, IMLER, J.L. ET AL. 'An efficient procedure to select and recover recombinant adenovirus vectors'
- NUCLEIC ACIDS RESEARCH, vol. 21, no. 15, 25 Juillet 1993 OXFORD GB, pages 3601-3602, BUBECK, P. ET AL 'Rapid cloning by homologous recombination in vivo'
- METHODS: A COMPANION TO METHODS IN ENZYMOLOGY, vol. 5, 1993 pages 161-175, SPENCER, F. ET AL. 'Targeted recombination-based cloning and manipulation of large DNA segments in yeast'
- NUCLEIC ACIDS RESEARCH, vol. 21, n°22, 1993 pages 5192-5197, OLINER, J.D. ET AL.'In vivo cloining of PCR products in E. coli'

## Description

La présente invention concerne une méthode pour préparer un vecteur viral *in vitro* dans une cellule procaryote et son application pour produire une particule virale infectieuse destinée à un usage thérapeutique et, en particulier, un usage en thérapie génique.

La possibilité de traiter les maladies humaines par thérapie génique est passée en quelques années du stade des considérations théoriques à celui des applications cliniques. Le premier protocole appliqué à l'homme a été initié aux Etats Unis en septembre 1990 sur un patient génétiquement immunodéficient en raison d'une mutation affectant le gène codant pour l'Adénine Désaminase (ADA). Le succès relatif de cette première expérimentation a encourage le développement de nouveaux protocoles de thérapie génique pour diverses maladies génétiques ou acquises (maladies infectieuses et notamment virales comme le SIDA ou cancers). La grande majorité des protocoles décrits jusqu'à présent met en oeuvre des vecteurs viraux pour transférer et exprimer le gène thérapeutique dans les cellules à traiter.

A ce jour, les vecteurs rétroviraux sont parmi les plus utilisés du fait de la simplicité de leur génome. Cependant, outre leur capacité restreinte de clonage, ils présentent deux inconvénients majeurs qui limitent leur utilisation systématique : d'une part, ils infectent majoritairement les cellules en division et d'autre part, du fait de leur intégration au hasard dans le génome de la cellule hôte, le risque de mutagénèse insertionnelle n'est pas négligeable. C'est pourquoi, de nombreuses équipes scientifiques se sont attachées à développer d'autres types de vecteurs, parmi lesquels on peut citer ceux issus des adénovirus, virus associés aux adénovirus (AAV), poxvirus et virus de l'herpès. D'une manière générales leur organisation et leur cycle d'infection sont largement décrits dans la littérature accessible à l'homme de l'art.

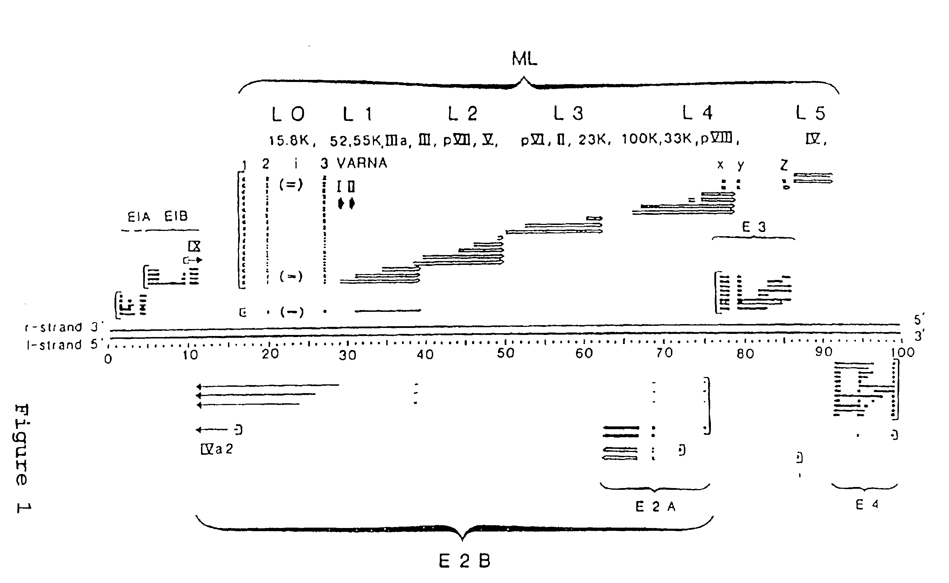

A cet égard, l'emploi des vecteurs adénoviraux est apparu comme une alternative prometteuse. Les adénovirus ont été mis en évidence dans de nombreuses espèces animales, ont un large spectre d'hôte, sont peu pathogènes et ne présentent pas les inconvénients liés aux rétrovirus puisqu'ils se répliquent dans les cellules quiescentes et sont non-intégratifs. A titre indicatif, leur génome est constitué d'une molécule d'ADN linéaire et bicaténaire d'environ 36 kb portant plus d'une trentaine de gènes, à la fois des gènes précoces nécessaires à la replication virale (E1 à E4) et des gènes tardifs de structure (L1 à L5) (voir Figure 1).

D'une manière générale, les vecteurs adénoviraux sont obtenus par délétion d'au moins une partie des gènes viraux (notamment de la région E1 essentielle à la replication virale) qui sont remplacés par les gènes thérapeutiques En conséquence, ils sont propagés dans une lignée cellulaire, dite de complémentation, qui fournit *en trans* les fonctions virales délétées pour générer une particule de vecteur viral défective pour la replication mais capable d'infecter une cellule hôte. On utilise couramment la lignée 293, établie à partir de cellules de rein embryonnaire humain, qui complémente les adénovirus défectifs pour la fonction E1 (Graham et al., 1977, J. Gen. Virol., 36, 59-72).

Les techniques de préparation des vecteurs adénoviraux sont largement décrites dans la littérature (voir notamment Graham et Prevec, Methods in Molecular Biology, Vol 7 ; Gene Transfer and Expression Protocols ; Ed : E.J. Murray, 1991, The Human Press Inc., Clinton, NJ). Une des méthodes les plus employées consiste à générer le vecteur adénoviral recombinant dans les cellules de complémentation transfectées avec un plasmide bactérien portant le gène d'intérêt sous-cloné au sein de sa région d'insertion adénovirale et le génome adénoviral. Généralement, ce dernier est clivé par une enzyme de restriction appropriée afin de réduire l'infectivité de l'ADN viral parental et augmenter l'efficacité d'isolement des adénovirus recombinants. Cependant, on observe néanmoins un bruit de fond important de particules virales infectieuses d'origine parentale, ce qui nécessite un travail d'analyse des plages obtenues particulièrement lourd (d'un point de vue temps et coût puisque chaque plage doit être amplifiée et analysée individuellement). Ceci est problématique lorsque le virus parental présente un avantage sélectif sur l'adénovirus recombinant, par exemple lorsque ce dernier se réplique plus lentement que le virus parental, du fait de l'insertion d'un gène d'intérêt de grande taille (Facteur VIII, dystrophine), réduisant d'autant sa probabilité d'obtention.

La ligation entre deux fragments d'ADN générés par les techniques classiques de biologie moléculaire et portant respectivement les parties 5' et 3' du vecteur adénoviral recombinant peut également être employée. La transfection du mélange de ligation dans les cellules de complémentation permet en théorie d'encapsider le génome de l'adénovirus recombinant pour former une particule infectieuse. Cette technologie est peu efficace et son application limitée par le nombre restreint de sites de restriction appropriés et uniques.

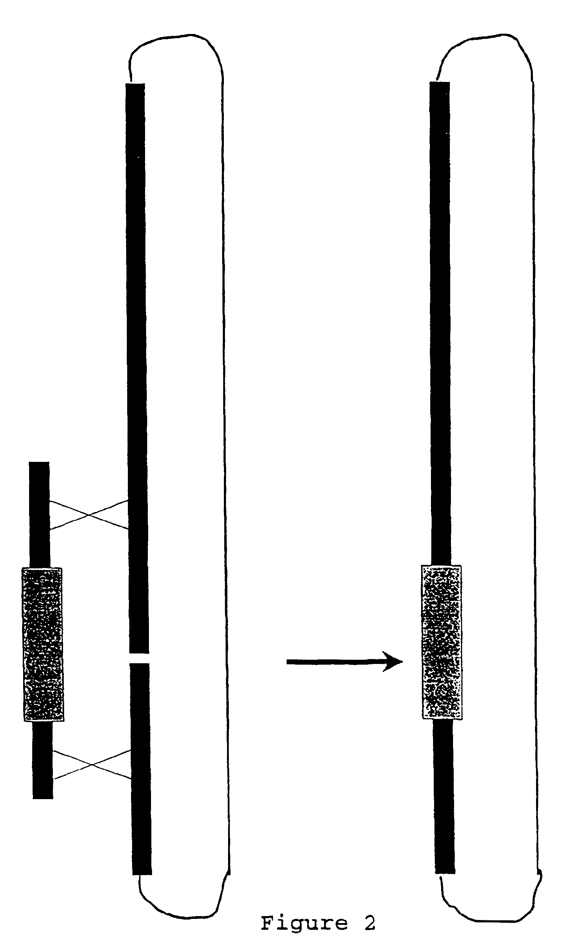

On a maintenant montré qu'il est possible de générer dans *Escherichia coli* (*E. coli*) rec BC sbc BC un vecteur adénoviral recombinant par recombinaison homologue intermoléculaire entre un plasmide contenant le génome d'un adénovirus de type 5 et un insert portant une séquence d'ADN exogène entourée de séquences adénovirales A et B (Figure 2). Ce procédé conduit au remplacement dans le génome adénoviral de la région ciblée située entre A et B par la séquence exogène. La transfection du vecteur adénoviral recombinant ainsi généré dans une lignée de complémentation adéquate donne lieu à une particule virale infectieuse qui peut être utilisée sans étape préalable de purification pour infecter une cellule hôte. Le bruit de fond (contamination par des particules virales parentales) est réduit voire même supprimé. On a trouvé de manière surprenante que l'usage de souches d'E. coli recBC sbcBC est particulièrement avantageux pour favoriser la recombinaison intermoléculaire entre deux fragments quelconques d'ADN.

Le procédé de la présente invention repose sur l'exploitation des activités enzymatiques endogènes des cellules procaryotes impliquées dans la recombinaison homologue, cette technique de recombinaison intermoléculaire avait déjà été décrite pour le clonage de petits inserts dans les vecteurs bactériens (Bubeck et al . 1993, Nucleic Acids Research, *21*, 3601-3602) ou pour générer par recombinaison intramoléculaire des gènes hybrides (Calogero et al., 1992, FEMS Microbiology Letters, *97*, 41-44 ; Caramori et al., 1991, Gene, *98*, 37-44). Cependant, cette technologie de recombinaison n'avait jamais été mise en oeuvre dans des cellules procaryotes pour générer des vecteurs viraux infectieux (capables d'être encapsidés en particules virales infectieuses).

Le procédé de l'invention présente l'avantage sur les techniques antérieures d'être rapide, particulièrement efficace et de ne requérir que peu de manipulations *in vitro*. De plus, il peut être mis en oeuvre avec des fragments d'ADN générés par PCR (Polymerase Chain Réaction) évitant ainsi les étapes de sous-clonage de la séquence d'ADN exogène dans la région d'insertion du génome viral. Enfin, il apporte une solution avantageuse au problème de bruit de fond clairement identifié dans la littérature. Par conséquent, il s'avère particulièrement performant pour les vecteurs issus de virus de grande taille ou dans lesquels on envisage d'insérer une séquence d'ADN exogène de grande taille.

C'est pourquoi la présente invention a pour objet un procédé pour préparer dans une cellule procaryote un vecteur viral recombinant d'au moins 20 kb dérivé d'un virus parental dans le génome duquel est insérée une séquence d'ADN exogène, par recombinaison intermoléculaire entre (i) un premier fragment d'ADN comprenant tout ou partie dudit génome du virus parental et (ii) un second fragment d'ADN comprenant ladite séquence d'ADN exogène entourée de séquences flanquantes A et B homologues à (i), caractérisé en ce que la cellule procaryote est dérivée d'une souche d'Escherichia coli rec BC sbc BC.

Bubeck et al., Nucleic Acids Research, vol 21, n° 15, 25 juillet 1993, pp. 3601-3602 : « Rapid cloning by homologous recombination in vivo », décrivent des techniques de clonage permettant d'obtenir des plasmides recombinants (vecteurs de clonage) utilisables dans le cadre de techniques de biologie moléculaire et reposant notamment sur l'introduction dans lesdits plasmides de fragments d'ADN de petites tailles (variant de 0,3 à 1,8 kb). A la même époque, Oliver et al., Nucleic Acids Research, vol 21, 1993, pp. 5192-5197 publiaient des travaux comparables et reconnaissaient que l'efficacité du clonage de fragments d'ADN issus de la PCR était inversement proportionnelle à la taille desdits fragments.

La présente invention s'avère particulièrement avantageuse lorsqu'il s'agit de préparer un vecteur viral recombinant d'au moins 20 kb et plus particulièrement d'un vecteur ayant un génome d'une longueur de 80 à 120% de celle du génome du virus sauvage correspondant, notamment de 90 à 110% et, de préférence, de 95 à 105%.

Au sens de la présente invention, un vecteur viral recombinant est obtenu à partir d'un virus parental modifié de manière à porter en un site approprié du génome viral et exprimer une séquence d'ADN exogène. Les virus parentaux susceptibles d'être utilisés dans le cadre de l'invention sont, par exemple, les alphavirus, les rétrovirus, les poxvirus et notamment le virus de la vaccine ou le canarypox les virus de l'herpès, les virus associés aux adénovirus (AAV) et tout particulièrement les adénovirus.

A cet égard, le choix de l'adénovirus parental est très large. Il peut s'agir d'un adénovirus d'origine humaine, canine, aviaire, bovine, murine, ovine, porcine ou simienne ou encore d'un adénovirus hybride comprenant des fragments de génome adénoviral de différentes origines. On préférera tout particulièrement un adénovirus d'origine humaine de sérotype C et de préférence un adénovirus de type 2 ou 5 ou encore un adénovirus d'origine animale de type CAV-1 ou CAV-2 (canine), DAV (aviaire) ou encore BAd (bovine). Ces virus et leur génome sont décrits dans la littérature (voir par exemple Zakharchuk et al., 1993, Arch. Virol., *128*, 171-176 ; Spibey et Cavanagh, 1989, J. Gen. Virol., *70*, 165-172 ; Jouvenne et al., 1987, Gene, *60*, 21-28 ; Mittal et al., 1995, J. Gen. Virol., *76*, 93-102).

Un procédé selon l'invention a pour objet la préparation d'un vecteur viral recombinant pour le transfert et l'expression d'une séquence d'ADN exogène dans une cellule hôte. Par "séquence d'ADN exogène", on entend un acide nucléique qui comprend des séquences codantes et des séquences régulatrices permettant l'expression desdites séquences codantes et dans lequel les séquences codantes sont des séquences qui ne sont normalement pas présentes dans le génome d'un virus parental en usage dans la présente invention ou si elles le sont dans un contexte génomique différent. Dans le cadre de l'invention, la séquence d'ADN exogène peut-être constituée d'un ou plusieurs gènes. Les séquences régulatrices peuvent être d'origines quelconques.

D'une manière préférée, la séquence d'ADN exogène peut coder pour un ARN anti-sens et/ou un ARNm qui sera ensuite traduit en protéine d'intérêt. Elle peut être de type génomique, de type ADN complémentaire (ADNc) ou de type mixte (minigène, dans lequel au moins un intron est délètè). Elle peut coder pour une protéine mature, un précurseur d'une protéine mature, notamment un précurseur destiné à être sécrété et comprenant de ce fait un peptide signal, une protéine chimérique provenant de la fusion de séquences d'origines diverses ou un mutant d'une protéine naturelle présentant des propriétés biologiques améliorées ou modifiées. Un tel mutant peut être obtenu par mutation, délétion, substitution et/ou addition d'un ou plusieurs nucléotide(s) du gène codant pour la protéine naturelle.

Dans le cadre de la présente invention, il peut être avantageux d'utiliser :
- les gènes codant pour des cytokines ou des lymphokines, comme les interférons α, β et γ, les interleukines et notamment l'IL-2, l'IL-6 ou l'IL-10, les facteurs nécrosant des tumeurs (TNF) et les facteurs stimulateurs de colonies (CSF) ;
- les gènes codant pour des récepteurs cellulaires, comme les récepteurs reconnus par des organismes pathogènes (virus, bactéries, ou parasites), de préférence par le virus VIH (Virus de l'Immunodéficience Humain), ou les ligands de récepteurs cellulaires ;
- les gènes codant pour les hormones de croissance (HGF) ;
- les gènes codant pour des facteurs de coagulation, comme le facteur VIII et le facteur IX;
- le gène codant pour la dystrophine ou la minidystrophine ;
- le gène codant pour l'insuline ;
- les gènes codant pour des polypeptides intervenant directement ou indirectement dans les canaux ioniques cellulaires, comme la protéine CFTR (Cystic Fibrosis Transmembrane Conductance Regulator) ;
- les gènes codant pour des ARN anti-sens ou des protéines capables d'inhiber l'activité d'une protéine produite par un gène pathogène, présent dans le génome d'un organisme pathogène, ou par un gène cellulaire, dont l'expression est dérègulée, par exemple un oncogène ;
- les gènes codant pour un inhibiteur de protéase comme l'α1-antitrypsine ou un inhibiteur d'une protéase virale ;
- les gènes codant pour des variants de protéines pathogènes qui ont été mutées de façon à altérer leur fonction biologique, comme par exemple des variants trans-dominants de la protéine TAT du virus VIH capables de compétition avec la protéine naturelle pour la liaison à la séquence cible, empêchant ainsi la réplication du VIH ;
- les gènes codant pour des épitopes antigéniques afin d'accroître l'immunité de la cellule hôte ;
- les gènes codant pour des polypeptides ayant des propriétés anticancéreuses et notamment des suppresseurs de tumeurs comme la protéine p53 ;
- les gènes codant pour les protéines du complexe majeur d'histocompatibilité des classes I et II, ainsi que les gènes codant pour les protéines régulatrices de l'expression de ces gènes ;
- les gènes codant pour des enzymes cellulaires ou produites par des organismes pathogènes ; et
- les gènes suicides. On peut citer plus particulièrement le gène TK-HSV-1. L'enzyme TK virale présente une affinité nettement supérieure par rapport à l'enzyme TK cellulaire pour certains analogues de nucléosides (comme l'acyclovir ou le gancyclovir). Elle les convertit en molécules monophosphatées, elles-mêmes convertibles par les enzymes cellulaires, en précurseurs de nucléotides, qui sont toxiques Ces analogues de nucléotides sont incorporables dans les molécules d'ADN en voie de synthèse, donc principalement dans l'ADN des cellules en état de réplication Cette incorporation permet de détruire spécifiquement les cellules en division comme les cellules cancéreuses.
- les gènes codant pour un anticorps, un fragment d'anticorps ou une immunotoxine.
- les gènes rapporteurs comme le gène Lac-Z, codant pour la β-galactosidase ou le gène luciférase.

Cette liste n'est pas limitative et bien entendu d'autres gènes peuvent être également employés.

Par ailleurs, une séquence d'ADN exogène en usage dans la présente invention peut en outre comprendre un gène non-thérapeutique, par exemple un gène codant pour un marqueur de sélection permettant de sélectionner ou identifier les cellules hôtes transfectées. On peut citer le gène *néo* (codant pour la néomycine phosphotransférase) conférant une résistance à l'antibiotique G418, le gène *dhfr.* (Dihydrofolate Réductase), le gène CAT (Chloramphenicol Acetyl transférase), le gène pac (Puromycine Acétyl-Transferase) ou encore le gène *gpt* (Xanthine Guanine Phosphoribosyl Transferase).

Bien entendu, un gène en usage dans la présente invention peut être placé sous le contrôle d'éléments appropriés à son expression dans une cellule hôte. Par "éléments appropriés", on entend l'ensemble des éléments nécessaires à sa transcription en ARN (ARN anti-sens ou ARNm) et à la traduction d'un ARNm en protéine. Parmi les éléments nécessaires à la transcription, le promoteur revêt une importance particulière. Il peut s'agir d'un promoteur constitutif ou d'un promoteur régulable et il peut être isolé d'un gène quelconque d'origine eucaryote ou même virale Alternativement, il peut s'agir du promoteur naturel du gène en question D'une façon générale, un promoteur en usage dans la présente invention, peut être modifié de manière à contenir des séquences régulatrices. A titre d'exemples de promoteurs tissus-spécifiques, on peut citer ceux des gènes d'immunoglobulines lorsque l'on cherche à cibler l'expression dans des cellules hôtes lymphocytaires et du gène α 1-antitrypsine pour une expression foie-spécifique. On peut également mentionner les promoteurs constitutifs du gène TK-HSV-1 (thymidine kinase du virus de l'herpès de type 1), du gène PGK (Phospho Glycerate kinase) murin ou humain, le promoteur précoce du virus SV40 (Simian Virus 40), un LTR rétroviral, ou encore le promoteur adénoviral MLP, notamment de adénovirus humain de type 2.

Le procédé selon l'invention met en oeuvre un mécanisme de recombinaison homologue intermoléculaire. D'une manière générale, il consiste en l'échange de séquences homologues entre deux fragments d'ADN. Ces séquences peuvent être identiques ou substantiellement homologues. En d'autres termes le degré d'homologie des séquences A et B avec la partie correspondante du premier fragment d'ADN peut être variable mais doit être suffisant pour permettre la recombinaison intermoléculaire. Aux fins de la présente invention, il est préférable qu'il soit supérieur à 70%, avantageusement supérieur à 80%, de préférence supérieur à 90% et de manière tout à fait préférée aux environs de 100%. De plus, une courte région d'homologie peut être suffisante (au moins 10 nucléotides consécutifs communs entre les séquences A et B et leurs séquences homologues dans le premier fragment d'ADN). Dans le cadre de la présente invention, la longueur des séquences A et B est de préférence comprise entre 10 pb et 10 kb, avantageusement 20 pb et 5 kb, de préférence 30 pb et 2 kb et, de manière tout à fait préférée, 40 pb et 1 kb.

Selon un mode avantageux, un procédé selon l'invention conduit au remplacement du matériel génétique localisé entre les séquences du premier fragment d'ADN homologues aux séquences A et B par la séquence exogène. Cet échange intermoléculaire permet de générer un vecteur viral recombinant circulaire sous forme d'un vecteur procaryotique (plasmide, cosmide ou phage) permettant sa manipulation et/ou sa propagation dans la cellule procaryote Un mode de réalisation du mécanisme mis en oeuvre dans le cadre de la présente invention est illustré dans la Figure 2.

Bien que la région d'insertion de la séquence exogène puisse être située à n'importe quelle position du génome viral, on préfère éviter les régions agissant *en cis* nécessaires à la replication. Ces dernières comprennent notamment les LTRs 5' et 3' ainsi que le signal d'encapsidation dans le cas des rétrovirus et les ITRs 5'et 3'et la région d'encapsidation s'agissant des adénovirus On indique que la région d'insertion peut être dirigée en des positions variées en fonction des séquences homologues A et B choisies.

Comme indiqué précédemment, le premier fragment d'ADN comprend tout ou partie du génome du virus parental en usage dans le cadre de l'invention. Il peut s'agir du génome d'un virus sauvage ou du génome d'un virus en dérivant modifié par délétion, addition et/ou substitution d'un ou plusieurs nucléotides. En particulier, il peut être délété d'un ou plusieurs gènes en totalité ou en partie

Le premier fragment d'ADN est de préférence inclu dans un vecteur conventionnel Le choix de celui-ci est très large, mais on peut citer plus particulièrement le pBR322, le p polyII ou encore le p poly III*I (Lathe et al., 1987, Gene, *57*, 193-201). Selon un mode de réalisation avantageux du procédé selon l'invention, le premier fragment d'ADN est de préférence linéarisé au niveau de la région dans laquelle on envisage de cibler l'insertion. En particulier, il peut être clivé par une ou plusieurs enzymes de restriction dont les sites sont localisés entre les séquences du premier fragment d'ADN homologues aux séquences A et B, mais également au niveau de ces dernières bien que ce mode de réalisation ne soit pas préféré. Le choix des sites de restriction à utiliser selon le virus parental retenu est à la portée de l'homme du métier. Avantageusement, on préférera utiliser des sites peu représentés dans le premier fragment d'ADN et en particulier uniques. Par ailleurs, de tels sites peuvent également être crées par les techniques classiques de mutagenèse dirigée.

Le second fragment d'ADN en usage dans la présente invention porte notamment la séquence d'ADN exogène entourée des séquences A et B impliquées dans la recombinaison intermoléculaire On préfère employer un fragment d'ADN linéaire. Il peut être généré par PCR, excisé d'un vecteur quelconque ou produit par voie synthétique ou toute méthode conventionnelle dans le domaine de l'art. A titre d'exemple, un second fragment d'ADN en usage dans le cadre de la présente invention peut être obtenu par amplification de la séquence d'ADN exogène à partir d'un vecteur de l'art antérieur ou d'une banque génomique ou d'un ADNc adéquat à l'aide d'amorces appropriées munies à leurs extrémités 5' des séquences A et B. En outre, un second fragment d'ADN peut également comprendre des séquences plasmidiques à ses extrémités 5'et 3', qui peuvent éventuellement être utilisées à titre de séquences A et B dans la mesure où elles sont homologues aux séquences plasmidiques contenues dans le premier fragment d'ADN.

Selon un mode de réalisation particulier, un procédé selon l'invention est mis en oeuvre pour la préparation d'un vecteur viral recombinant défectif pour la replication. Ce terme désigne un vecteur incapable d'achever un cycle infectieux autonome dans une cellule hôte. Généralement, le génome de ces vecteurs défectifs est dépourvu d'un ou plusieurs gènes essentiels à la replication, gènes précoces et/ou gênes tardifs. Ils peuvent être délétés en totalité ou en partie ou rendus non fonctionnels par mutation (addition, délétion et/ou substitution d'un ou plusieurs nucléotides). A cet égard, un procédé selon l'invention peut permettre la préparation d'un vecteur adénoviral recombinant dépourvu de tout ou partie des régions E1, E2, E3 et/ou E4.

A titre illustratif, on peut citer les modes de réalisation suivants. Lorsqu'il s'agit de préparer dans une cellule procaryote un vecteur adénoviral recombinant dérivant d'un adénovirus humain de type 5 (Ad5) dans lequel on envisage d'insérer une séquence d'ADN exogène à la place de la région E1, on peut mettre en oeuvre (i) un vecteur comportant le génome de l'Ad5 clivé par l'enzyme *Cla*I (site de restriction situé en position 918 du génome Ad5 tel que divulgué dans la banque de données Genebank sous la référence M73260) et (ii) un fragment d'ADN comprenant une séquence A homologue à la région d'encapsidation adénovirale, la séquence d'ADN exogéne suivie d'une séquence B homologue à la séquence codant pour la protéine pIX. Par ailleurs, l'usage d'un vecteur ponant le génome adénoviral clivé par l'enzyme *Spe*I (position 27082 du génome Ad5) et d'un fragment d'ADN comprenant une séquence A homologue à l'extrémité 5' de la région E2, la séquence d'ADN exogène et une séquence B homologue à l'extrémité 3' de la région E4 permettra de préparer un vecteur adénoviral recombinant dans lequel la séquence d'ADN exogène est insérée à la place de la région E3. Bien entendu, ces modes de réalisation particuliers ne sont cités qu'à titre d'exemples. Enfin, le procédé selon l'invention peut être utilisé pour introduire des délétions, mutations et/ou substitutions dans une région particulière d'un génome viral.

Selon un autre mode de réalisation, un procède selon l'invention peut également être employé pour insérer au moins deux fragments d'ADN au sein du génome viral, par recombinaison intermoléculaire entre (i) un premier fragment d'ADN comprenant tout ou partie dudit génome du virus parental, (ii) un second fragment d'ADN comprenant une première partie de ladite séquence d'ADN exogène munie à son extrémité 5' de ladite séquences flanquantes A et (iii) un troisième fragment d'ADN comprenant une deuxième partie de ladite séquence d'ADN exogène munie à son extrémité 3' de ladite séquences flanquantes B ; lesdits seconds et troisièmes fragments d'ADN comportant une séquence homologue se recouvrant à leurs extrémités 3' et 5' respectives. A titre indicatif, ces séquences homologues entre les seconds et troisièmes fragments d'ADN répondent aux mêmes critères d'homologie et de longueur que les séquences A et B. Ce mode de réalisation spécifique est particulièrement avantageux pour le clonage des séquences exogènes de grande taille.

Le procédé selon l'invention est mis en oeuvre dans une bactérie dérivée d'une souche *d'Escherichia coli* recBC sbcBC comme par exemple les souches CES200, CES201, W5449 et BJ5183 (Hanahan, 1983, J. Mol. Biol., *166*, 557-580).

Une procédure typique d'un procédé selon l'invention comprend les étapes suivantes :
(a) on co-introduit ou on introduit de manière séparée dans une cellule un premier fragment d'ADN et (ii) un second fragment d'ADN tels que définis précédemment,
(b) on cultive la cellule obtenue à l'étape (a) dans des conditions appropriées pour permettre la génération du vecteur viral recombinant par recombinaison intermoléculaire, et
(c) on récupère le vecteur viral recombinant.

Dans le cadre d'un procédé selon l'invention, les quantités de premiers et seconds fragments d'ADN peuvent varier. On préfère employer une concentration 10 fois plus importante de second fragment par rapport au premier. L'introduction des fragments d'ADN dans une cellule d'Escherichia coli rec BC sbc BC et la récupération du vecteur de ces mêmes cellules sont réalisées selon les techniques générales de génie génétique et de clonage moléculaire détaillées dans Maniatis et al. (1989, Laboratory Manual, Cold Spring Harbor, Laboratory Press, Cold Spring Harbor, NY).

Une particule virale infectieuse contenant un vecteur viral recombinant peut être obtenu en mettant en oeuvre un procédé selon lequel :
(a) on introduit ledit vecteur viral recombinant dans une cellule mammifère pour générer une cellule de mammifère transfectée,
(b) on cultive ladite cellule de mammifère transfectée dans des conditions appropriées pour permettre la production de ladite particule virale, et
(c) on récupère ladite particule virale de la culture cellulaire obtenue à l'étape (b).

Les cellules peuvent être transfectées selon les techniques standards bien connues de l'homme du métier. On peut citer notamment la technique au phosphate de calcium, la technique au DEAE dextran, l'électroporation, les méthodes basées sur les chocs osmotiques, la microinjection ou les méthodes basées sur l'emploi de liposomes. Selon un mode de réalisation préféré, la cellule mammifère est avantageusement une cellule de complémentation et notamment la cellule 293 dans le cadre d'un vecteur dérivant d'un adénovirus Les particules virales peuvent récupérees du surnageant de culture, mais également des cellules selon les protocoles conventionnels.

Une particule virale infectieuse ou un vecteur viral recombinant préparé selon un procédé selon l'invention peut être utilisé pour le traitement thérapeutique ou chirurgical du corps humain et notamment par thérapie génique. Ledit traitement est plus particulièrement un traitement préventif ou curatif de maladies telles que les maladies génétiques (hémophilie ; thalassémies, emphysème, maladie de Gaucher, mucoviscidose, myopathie de Duchène ou de Becker...) les cancers, les maladies virales (SIDA., infections herpétiques ou dues au cytomégalovirus ou au papillomavirus). Les vecteurs et particules virales peuvent être introduits soit *in vitro* dans une cellule hôte prélevée du patient, soit directement *in vivo* dans l'organisme à traiter. De préférence, la cellule hôte est une cellule humaine et, de manière préférée une cellule pulmonaire, fibroblastique, musculaire, hépatique, lymphocytaire ou une cellule de la lignée hématopoïétique.

Une composition pharmaceutique comprenant une quantité thérapeutiquement efficace d'une particule virale infectieuse ou d'un vecteur viral recombinant prépare selon un procédé selon l'invention peut être préparée en association avec un véhicule acceptable d'un point de vue pharmaceutique Une telle composition pharmaceutique peut être préparée selon les techniques communément en usage et administrée par toute voie d'administration connue, par exemple parvoie systémique (notamment intraveineuse, intratrachéale, intrapéritonéale, intramusculaire, sous-cutanée intratumorale ou intracranienne) ou par aérosolisation ou instillation intrapulmonaire.

Une particule virale infectieuse ou un vecteur viral recombinant préparé selon un procédé selon l'invention peut être utilisé pour l'expression d'une séquence d'ADN d'intérêt dans un système cellulaire.

La présente invention est plus complètement décrite en référence aux figures suivantes et à l'aide des exemples suivants.

La Figure 1 est une représentation schématique du génome de l'adénovirus humain de type 5 (représenté en unités arbitraires de 0 à 100), indiquant l'emplacement des différents gènes.

La Figure 2 illustre un procédé de recombinaison intermoléculaire entre deux fragments d'ADN. les séquences plasmidiques sont représentées par un trait fin, les séquences virales par un trait gras et la séquence exogène par une boite hachurée.

La Figure 3 est une représentation schématique du vecteur pTG3614 correspondant au p polyII dans lequel est cloné le génome d'un adénovirus recombinant modifié dans la région E1 par insertion d'une cassette d'expression du gène FIX humain.

La Figure 4 est une représentation schématique du vecteur pTG4652 correspondant au p polyII dans lequel est cloné le génome d'un adénovirus recombinant modifié dans les régions E1, E3 et E4 par insertion d'une cassette d'expression du gène CF humain et délétions partielles.

### EXEMPLES

Les exemples suivants n'illustrent qu'un mode de réalisation de la présente invention.

Les constructions décrites ci-après sont réalisées selon les techniques générales de génie génétique et de biologie moléculaire détaillées dans Maniatis et al (1989, *supra*). Les sites de restriction protubérants en 5' peuvent être convertis en sites francs par traitement par le grand fragment Klenow de l'ADN polymérase d'*E.coli* alors que les sites protubérants en 3' sont traités par la T4 polymérase. En ce qui concerne les étapes d'amplification par PCR, on applique le protocole tel que décrit dans PCR Protocols-A guide to methods and applications (1990, ed Innis, Gelfand, Sninsky et White, Academic Press Inc). Les cellules sont transfectées par les méthodes conventionnelles et sont cultivées selon les recommandations du fournisseur. Les fragments insérés dans les différentes constructions décrites ci-après sont indiqués précisément selon leur position dans le génome de l'Ad5 telle que divulguée dans la banque de données Genebank sour la référence M73260.

### EXEMPLE 1 : Construction d'un vecteur viral recombinant dérivé d'un adénovirus humain de type 5 sous forme d'un plasmide bactérien

### A. Clonage du génome d'Adénovirus 5 (Ad5) dans p polyII

On synthétise l'extrémité gauche du génome d'Ad5 (nucléotides 1 à 935) par PCR à l'aide des amorces oligonucléotidiques oTG6597(SEQ ID NO: 1) et oTG6598 (SEQ ID NO: 2) La première s'hybride aux 21 premiers nucléotides de l'ITR 5' et présente un site *Pac*I, immédiatement en amont des séquences virales, et un site *Eco*RI utilisé pour le clonage. La deuxième amorce permet l'introduction des sites *Sal*I puis *Bgl*II en aval des séquences virales La matrice engagée dans cette réaction est l'ADN genomique d'Ad5 préparé selon les méthodes conventionnelles On digère le fragment ainsi amplifié par *Eco*RI et *Bg*III et on le clone dans le plasmide p polyII (Lathe et al., 1987. *supra)* préalablement clivé par ces deux mêmes enzymes de restriction pour obtenir le pTG1692.

On prépare le fragment d'ADN correspondant à l'extrémité droite du génome d'Ad5 (nucléotides 35103 à 35935) suivant le même principe en utilisant le couple d'oligonucléotides oTG6599 (SEQ ID NO: 3) et oTG6597 (SEQ ID NO: 1). On construit le pTG1693 en insérant le fragment amplifié digéré par *Eco*RI et *Bgl*II dans les mêmes sites de p polyII. On excise de pTG1693 un fragment *Bgl*II-*Bam*HI portant les séquences amplifiées de façon à l'introduire dans le site *Bgl*II de pTG1692 en aval des séquences 5' adénovirales Le pTG3601 ainsi obtenu est linéarisé entre les extrémités gauche et droite du génome d'Ad5 pardigestion avec les enzymes de restriction *Bgl*II ou *Sal*I. Les extrémités ainsi générées sont rendues franches par l'action du fragment klenow de l'ADN polymérase I d'*E*. *coli*. Des bactéries BJ5183 (Hanahan, 1983 *supra*) rendues compétentes par un traitement au chlorure de calcium sont co-transformées avec la préparation précédente et l'ADN génomique d'Ad5. Les colonies obtenues sont analysées par cartographie de restriction. On sélectionne un clone désigné pTG3602 généré par recombinaison homologue intermoléculaire dans lequel les séquences adénovirales (nucléotides 936 à 35102) ont été insérées entre les deux fragments produits par PCR, de manière à générer un vecteur plasmidique comprenant le génome complet de l'Ad5 - pTG3602 est produit dans les bactéries C600 (Huynh et al., 1985 DNA cloning, Volume 1, ed. Glover, IRL Press Limited : Oxford, England p56-110).

### B. Evaluation du pouvoir infectieux de p TG3602

On libère le génome viral par l'action de l'enzyme de restriction Pad. Des cellules 293 cultivées en monocouche sont transfectées avec le produit de cette digestion par la technique de précipitation au phosphate de calcium. On peut également utiliser d'autres cellules sensibles, par exemple les cellules A549 (ATCC CCL185). On fait croître les cellules sous agar pendant 9 à 14 jours, période durant laquelle l'apparition de plages de lyse sur le tapis cellulaire témoigne de la production de particules virales infectieuses et donc, de la capacité du génome d'Ad5 issu de pTG3602 à se répliquer.

### C. Construction d'un vecteur adénoviral recombinant défectif dans lequel le gène exogène remplace la région précoce E1.

On utilise un plasmide dans lequel le gène rapporteur Lac Z codant pour la β-galactosidase est placé dans un contexte viral ; par exemple un plasmide dérivé du pMLP-Adk7 (Stratford-Perricaudet et Perricaudet, 1991, Human Gene Transfer, 219, 51-61) comprenant les séquences 5' de l'Ad 5 (nucléotides 1 à 458), le promoteur MLP Ad2, le gène Lac Z, le signal de polyadénylation du virus SV40 et les séquences Ad5 comprises entre les nucléotides 3329 à 6241.

La cassette d'expression du gène Lac Z ceinte de séquences adénovirales est excisée par l'action des enzymes de restriction *Bsr*GI (position 192 sur le génome d'Ad5) et *Pst*I (position 3788) puis purifiée. Le pTG3602 est linéarisé au niveau du site *Cla*I (position 918) puis traité avec la klenow Les deux fragments obtenus sont co-transformés dans des bactéries BJ5183 compétentes. Une recombinaison au niveau des séquences adénovirales homologues entraîne le remplacement de la région E1 d'Ad5 (nucléotides 459 à 3328) par la cassette d'expression du gène rapporteur dans le plasmide pTG3603.

Son pouvoir infectieux est vérifié par translection d'un tapis de cellules 293 transfectées avec pTG3603 préalablement digéré par *Pac*I. Les plages de lyse formées sont alors piquées et les particules virales remises en suspension et utilisées pour infecter une monocouche de cellules 293. La coloration des cellules en bleu après addition de X-Gal témoigne de l'expression du gène Lac Z.

### D. Construction d'un vecteur adénoviral recombinant dans lequel le gène exogène remplace la région précoce E3

Une cassette d'expression du gène codant pour la protéine gp19 de la région E3 d'Ad5 (nucléotides 28731 à 29217) est assemblée dans le bactériophage M13mp18 (Gibco BRL) par clonage de deux fragments PCR l'un correspondant au LTR 3' de RSV (Rous Sarcoma Virus) (amorces oligonucléotidiques oTG5892-SEQ ID NO: 4 et 5893-SEQ ID NO: 5) et l'autre à la séquence codant pour la gp19 (oTG5455-SEQ ID NO: 6 et 5456-SEQ ID NO: 7). On obtient le vecteur M13TG1683, dont on excise la cassette d'expression par une digestion *Xba*I. Après traitement avec la klenow, il est introduit dans le site *Bam*I (rendu franc par action de l'ADN polymérase du phage T4) de pTG8519. Celui-ci dérive du plasmide puc19 (Gibco BRL), dans lequel on a inséré les séquences adénovirales comprises entre le site *Spe*I et l'extrémité droite du génome (nucléotides 27082 à 35935) mais dépourvues de la majorité de la région E3 (nucléotides 27871 à 30758). On obtient le pTG1695 dont on remplace le fragment *Sca*I-*Spe*I, porteur des séquences plasmidiques, par un fragment équivalent purifié de pTG1659. Celui-ci correspond au pucI9 comprenant les séquences de l'Ad5 s'étendant des nucléotides 21562 à 35826. Le pTG1697 ainsi obtenu présente des séquences adénovirales qui s'étendent du site *Bam*HI (position 21562) à l'ITR 3' (position 35935) dans lesquelles la région E3 est remplacée par une cassette d'expression de la gp19 sous contrôle du promoteur constitutif RSV Le fragment *Dra*I (position 22444 et 35142 sur le génome d'Ad5) est purifié, et co-introduit dans les bactéries BJ-5183 compétentes avec pTG3602 linéarisé par *Spe*I et traité par la klenow. Les recombinants porteurs d'un plasmide généré par recombinaison sont criblés pour la présence du promoteur RSV. Le pTG3605, vecteur plasmidique porteur du génome d'Adgp19+, est ainsi mis en évidence.

Le pouvoir infectieux génome viral excisé du plasmide pTG3605 est testé suivant le protocole déjà décrit précédemment. La production d'une protéine gp19 fonctionnelle est suivie par co-immunoprécipitation des antigènes du complexe majeur d'histocompatibilité de classe I et de celle-ci (Burgert et Kvist, 1985, Cell, *41*, 987-997).

### E. Construction d'un vecteur adénoviral recombinant défectif dans lequel les deux régions précoces E1 et E3 sont remplacées par des gènes exogènes

Les cellules BJ-5183 sont co-transformées par le fragment *Dra*I isolé de pTG1697 mentionné ci-dessus et le fragment *Spe*I (Klenow) isolé de pTG3603. Le plasmide résultant est testé dans les mêmes conditions que précédemment.

### F. Construction d'un vecteur adénoviral recombinant exprimant le gène FIX

Le cDNA codant pour le FIX humain a été cloné sous forme d'un fragment *Bam*HI dans le plasmide pTG381. Dans ce dernier, l'extrémité 5' non codante du gène FIX a été modifiée de manière à être conforme aux règles de Kozak. Le plasmide pTG381 est décrit dans la publication du brevet FR 2 600 334. Le gène FIX est recloné dans le site *Bam*HI de pTG6512, pour donner pTG4375. pTG6512 dérive du vecteur pTG6511 après délétion du promoteur tardif majeur (MLP) de l'Ad2 et remplacement par un polylinker. A titre indicatif, la construction du pTG6511 est détaillée à l'exemple 1 de la demande internationale WO 94/28152. Le promoteur PGK murin est isolé par les méthodes conventionnelles à partir d'ADN génomique de souris selon Adra et al. (1987, Gene *60*, 65-74) ou à l'aide d'amorces adéquates créées à partir des données de séquence divulguées dans la banque de données Genebank sous la référence XI5339. Des sites de restriction *Pst*I sont inclus aux extrémités 5' des amorces afin de faciliter les étapes de clonage ultérieures. Le fragment portant le promoteur PGK est traité par la T4 polymérase puis introduit en amont du gène humain FIX dans le site *Hpa*I de pTG4375 dont on excise par digestion *Msc*I un fragment dit "de remplacement" comportant la cassette FIX entourée de séquences adénovirales E1 (185pb en 5' et 2045pb en 3').

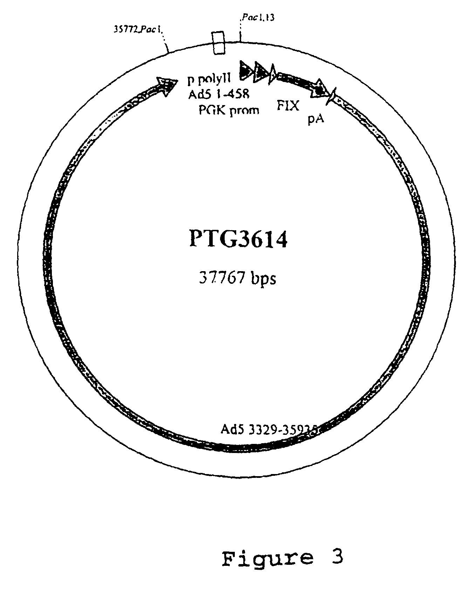

Celui-ci est cotransformé dans le bactéries BJ5183 en présence du vecteur pTG3602 digéré par *Cla*I (position 918). Les transformants sont sélectionnés sur ampicilline et analysés par cartographie de restriction On retient le clone désigné pTG3614 correspondant à un vecteur adénoviral comportant le gène thérapeutique FIX à la place de la région E1 (Figure 3). Un stock viral est constitué de manière conventionnelle, par transfection du génome adénoviral libéré par digestion *Pac*I dans la lignée 293. Après une étape d'amplification, les cellules sont lysées et les particules virales AdTG3614 purifiées par centrifugation sur chlorure de césium. La capacité à transférer et exprimer le gène FIX est évaluée en modèle animal souris. Pour ce faire, 1,5 x 10⁹ ufp sont injectées dans la veine caudale de souris femelles C57 Black 6 âgées de 5 à 6 semaines. Des échantillons de sang sont prélevés régulièrement sur lesquels on dose la quantité de FIX humain par ELISA (Kit Diagnostica Stago, Asnières, France ; Asserachirom ^{R} IX : Ag 00564). Le FIX est detecté dans le serum de souris pendant plusieurs semaines avec un maximum à 5 jours.

### G. Construction d'un vecteur adénoviral modifié dans la région E2

Lors du cycle viral normal, l'expression des gènes de la région E2 est induite par les produits précoces de E1 et E4 et on observe une production abondante des protéines codées par E2 et, en particulier, de la protéine DBP (pour DNA Binding Protein). Or, cette expression élevée peut être problématique *in vivo* (induction de réponses inflammatoires ou interférence avec l'expression du transgéne). C'est pourquoi, des vecteurs adénoviraux défectifs pour la fonction E2A ont été construits soit par introduction d'une mutation thermosensible dans E2A (position 22795 ; C en T résultant en un changement Pro en Ser) ou par d'une délétion partielle du gène DBP

Le vecteur pTG9551 portant la mutation thermosensible est généré par recombinaison homologue dans les cellules BJ entre pTG3601 digéré par *Bgl*II et l'ADN génomique purifié du virus Ad5Hts 125 (Ensinger et Ginsberg, 1972, J. Virol. 10, 328-339). Le génome ainsi reconstitué est libéré par digestion *Pac*I et tranfecté dans les cellules 293 qui sont incubées à 32°C pour la production des particules virales (la température non permissive est 39°C).

La défectuosité de E2A peut également être générée par délétion partielle des régions codantes du gène DBP en évitant les régions recouvrant d'autres cadres de lecture. La construction est réalisée par recombinaison homologue entre pTG3601 *Bgl*II et l'ADN viral préparé à partir du virus H5d1802 (Rice et Klessig, 1985, J.Virol. 56, 767-778). Les virus peuvent être produits dans une lignée de complementation appropriée trans-complémentant la fonction DBP, par exemple la lignée,293 transfectée par un vecteur permettant l'expression constitutive ou régulée du gène DBP.

### H. Construction d'un vecteur adénoviral modifié dans la région E4

Dans un premier temps, on construit un vecteur dit "de remplacement" comprenant une cassette d'expression du gène codant pour la protéine CFTR placée au sein de la région adénovirale E1. Ce vecteur désigné pTG8585 contient les éléments suivants, tous décrits dans la littérature :
- l'ITR 5' Ad5 (positions 1 à 458),
- le promoteur MLP Ad2,
- le cDNA CFTR humain,
- le signal de polyadenylation du gène de la β-globine de lapin,
- le LTR 3' du virus RSV (Rous Sarcoma virus),
- les séquences adénovirales codant pour la protéine gp 19 (positions 28731 à 29217 de l'Ad5), et
- la partie 3' de la région E1B (position 3329 à 6241).

En parallèle, les séquences adénovirales recouvrant les régions E3 et E4 sont sous-clonées dans le vecteur p poly Il et déletées par les techniques classiques de biologie moléculaire. On élimine les séquences E3 (positions 28592 à 30470) par digestion *Xba*I et les séquences E4 (positions 32994 à 34998). Le génome adénoviral ainsi modifié est reconstituté par recombinaison homologue entre un fragment de remplacement portant ces modifications isolé du vecteur précédent et pTG3603 digéré par *Spe*I. On obtient pTG8595 portant un génome adénoviral recombinant (gène Lac Z à la place de la région E1) et défectif pour les fonctions E3 et E4.

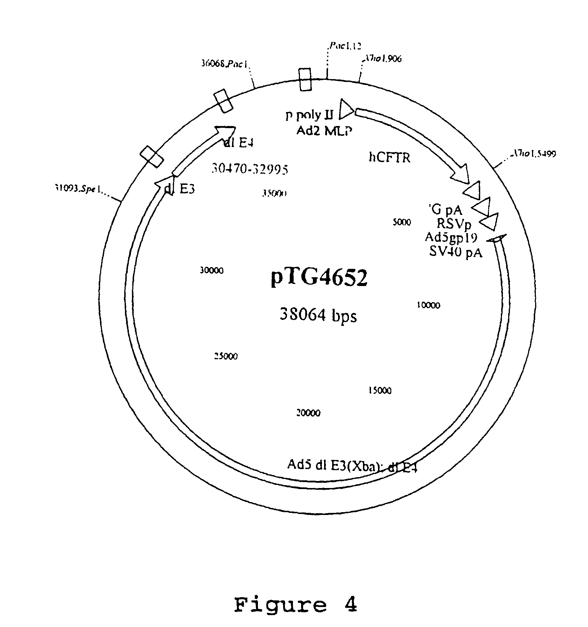

Les cassettes d'expression des gènes CFTR et gp19 indiquées ci-dessus sont introduites dans pTG8595 en remplacement du géne Lac Z par co-transformation des cellules BJ5183 par le fragment *Pac*I *- Bst*EII isolé de pTG8585 et le vecteur pTG8595 linéarisé par *Cla*I. On génère pTG4652 (Figure 4) qui peut être propagé dans une lignée cellulaire complémentant les fonctions E1 et E4 défectives telles que décrites dans la demande internationale WO94/28152.

Les particules virales sont amplifiées, les cellules lysées et les extraits cellulaires totaux sont analysés pour l'expression du gène CFTR par Western blot (gel SDS-PAGE 8 %). Les filtres sont incubés en présence d'une dilution au 1/5000 de l'anticorps monoclonal MAB 1104 dirigé contre un peptide synthétique correspondant aux acides aminés 722 à 734 de la protéine CFTR humaine. La détection est réalisée par la méthode ECL (Enhanced ChemiLuminescence ; kit Amersham). On observe dans les extraits de cellules infectées par AdTG4652 une bande de forte intensité et assez diffuse correspondant à un produit de poids moléculaire élevé comigrant avec un témoin CFTR. Cette bande n'est pas présente dans les extraits issus du virus parental pTG8595 qui ne contient pas la cassette d'expression CFTR.

### EXEMPLE 2 : Construction d'un vecteur viral recombinant dérivé de l'Adénovirus canin 2 (CAV2) sous forme d'un plasmide bactérien

### A. Clonage du génome de CAV2 dans p poly II

Le génome de CAV 2 est cloné dans ppolyII SfiI-NotI 14 (Lathe et al., 1987, *supra*) et modifié pour générer des vecteurs recombinants en utilisant les sites adéquats suivant les mêmes procédés que ceux utilisés dans le cas de la manipulation d'Ad5. Les extrémités gauche (nucléotides 1 à 810 suivant la numérotation de la séquence D04368 de Genebank) et droite (nucléotides -27600 à la fin) sont synthétisées par PCR entre les couples d'amorces oligonucléotidiques oTG6974 (SEQ ID NO: 8) et oTG6975 (SEQ ID NO: 9) et oTG6976 (SEQ ID NO: 10) et oTG6974, respectivement. Elles sont assemblées par l'intermédiaire du site *Pst*I introduit lors de la PCR à la suite des séquences virales, dans le cas de l'extrémité gauche, et cartographié à approximativement 1200 pb de la fin du genome, dans le cas de l'extémité droite. Des bactéries compétentes BJ5183 sont co-transformées avec le plasmide ainsi obtenu linéarisé par *Pst*I et l'ADN de CAV2. L'introduction des séquences virales manquantes (nucléotides 811 à 27600) est réalisée par recombinaison homologue. Le génome de CAV2 porté par le piasmide résultant est excisé par l'intermédiaire des sites *Not*I introduits lors de la PCR puis transfecté dans une monocouche de cellules MDCK (ATCC CCL34). Les étapes suivantes sont décrites dans l'exemple 1.

### B. Construction d'un vecteur adénoviral recombinant d'origine canine

L'ADN génomique de virus CAV2 (souche Toronto A 26/61 ; ATCC VR-800) est préparé par la technique classique (amplification sur lignée rénale de chien MDCK GHK ..., lyse des cellules, purification des virus par centrifugation sur chlorure de césium, traitement à la protéinase k et enfin extraction au phénol / chloroforme). Le génome CAV2, qui a une longueur de 31 kpb est introduit dans un vecteur plasmidique par recombinaison homologue. Pour ce faire, on isole les extrémités gauche et droite du génome CAV2 par PCR et digestion enzymatique en incorporant un site *Not*I immédiatement au-delà des ITRs 5' et 3'. Le vecteur pTG4457 est obtenu par introduction dans p poly II de la partie 5' du génome viral jusqu'au site *Bst*BI (position 870) suivie de la partie 3' à partir du site *Sal*I (position 27800). Le génome complet peut être reconstitué par cotransformation entre l'ADN génomique (100 à 500 ng) et pTG4457 digéré par *Bst*BI (10 à 100 ng). Le génome viral peut être excisé du vecteur précèdent pTG5406 par digestion *Not*I. On vérifie que l'ADN est infectieux par transfection de 1 à 5 µg dans les cellules de chien MDCK ou GHK. On observe la production de plages.

Le vecteur recombinant est obtenu de la façon suivante :

L'extrémité gauche du CAV2 est sous clonée et modifiée de manière à déleter les séquences codantes E1A en totalité et E1B partiellement Ceci est effectué par digestion *Nar*I puis réintroduction d'un fragment amplifié par PCR recouvrant la région d'encapsidation, la région promotrice E1A et le site d'initiation de la transcription Les amorces sont conçues de manière a intégrer également un site de restriction unique *Xba*I*.* On obtient pTG5407 dans lequel on introduit au niveau du site *Xba*I le gène CAT ou Lac Z (pTG5408). Ce dernier est digéré par *Xho*I et on insère la partie 3' du génome viral sous forme d'un fragment *Sal*I (position 27800 à la fin de l'ITR 3'). Le vecteur pTG5409 ainsi obtenu est linéarisé par *Sal*I et cotransformé dans *E.coli* BJ5183 avec le génome CAV-2 digéré par *Swa*I. On obtient un vecteur adénoviral d'origine canine portant le gène reporteur CAT à la place de la région E1.

## Revendications

1. Procédé pour préparer dans une cellule procaryote un vecteur viral recombinant d'au moins 20 kb dérivé d'un virus parental dans le génome duquel est insérée une séquence d'ADN exogène, par recombinaison intermoléculaire entre (i) un premier fragment d'ADN comprenant tout ou partie dudit génome du virus parental et (ii) un second fragment d'ADN comprenant ladite séquence d'ADN exogène entourée de séquences franquantes A et B homologues à (i)**caractérisé en ce que** ladite cellule procaryote est dérivée d'une souche d'*Escherichia coli* recBC sbcBC.

2. Procédé selon la revendication 1, **caractérisé en ce que** le virus parental est sélectionné parmi le groupe constitué par les adénovirus, les rétrovirus, les virus associés aux adénovirus, les poxvirus et les virus de l'herpès.

3. Procédé selon la revendication 2, **caractérisé en ce que** le virus parental est un adénovirus d'origine humaine, canine, aviaire, bovine, murine, ovine, porcine ou simienne ou encore un adénovirus hybride.

4. Procédé selon la revendication 3, **caractérisé en ce que** le virus parental est un adénovirus d'origine canine de type CAV-2.

5. Procédé selon la revendication 3, **caractérisé en ce que** le virus parental est un adénovirus d'origine humaine de sérotype C.

6. Procédé selon la revendication 5, **caractérisé en ce que** le virus parental est un adénovirus d'origine humaine de type 5.

7. Procédé selon l'une des revendications 1 à 6, **caractérisé en ce que** ladite séquence d'ADN exogène code pour un polypeptide d'intérêt thérapeutique sélectionné parmi le groupe constitué par les facteurs de coagulation, les hormones de croissance, les cytokines, les lymphokines, les polypeptides suppresseurs de tumeurs, les récepteurs cellulaires, les ligands de récepteurs cellulaires, les inhibiteurs de protéases, les anticorps, les toxines, les immunotoxines, la dystrophine et les polypeptides intervenant dans les canaux ioniques cellulaires tels que la protéine CFTR.

8. Procédé selon l'une des revendications 1 à 7, **caractérisé en ce que** les séquences flanquantes homologues A et B ont une longueur de 10pb à 10kb, avantageusement de 20 pb à 5kb, de préférence de 30 pb à 2 kb et de manière tout à fait préférée de 40 pb à 1kb.

9. Procédé selon l'une des revendications 1 à 8, **caractérisé en ce que** le premier fragment d'ADN est linéarisé au niveau de la région d'insertion de la séquence exogène.

10. Procédé selon l'une des revendications 1 à 9, pour la préparation d'un vecteur viral recombinant défectif pour la replication.

11. Procédé selon la revendication 10, pour la préparation d'un vecteur adénoviral recombinant dépourvu de tout ou partie d'au moins une région essentielle à la replication sélectionnée parmi les régions E1, E2 et E4.

12. Procédé selon la revendication 11, **caractérisé en ce que** le vecteur adénoviral recombinant est en outre dépourvu de tout ou partie de la région E3.

13. Procédé selon l'une des revendications 1 à 12, pour la préparation d'un vecteur viral recombinant d'au moins 30 kb.

14. Procédé selon les revendications 1 à 13, par recombinaison intermoléculaire entre (i) un premier fragment d'ADN comprenant tout ou partie dudit génome du virus parental, (ii) un second fragment d'ADN comprenant une première partie de ladite séquence d'ADN d'intérêt munie à son extrémité 5' de ladite séquence flanquante A et (iii) un troisième fragment d'ADN comprenant une deuxième partie de ladite séquence d'ADN d'intérêt munie à son extrémité 3' de ladite séquence flanquante B ; lesdits second et troisième fragments d'ADN comportant une séquence homologues à leurs extrémités 3' et 5' respectives.

15. Procédé selon l'une des revendications 1 à 14, pour introduire une modification par délétion, mutation et/ou substitution d'un ou plusieurs nucléotides ou une séquence d'ADN exogène dans un génome viral.

16. Usage d'une souche d'*E. coli* recBC sbcBC pour le clonage de fragments d'ADN dans un vecteur selon la revendication 1 par recombinaison homologue intermoléculaire.

## Patentansprüche

1. Verfahren zur Herstellung eines rekombinanten viralen Vektors von mindestens 20 kb in einer prokaryotischen Zelle, wobei der Vektor von einem Stammvirus abgeleitet wird, in dessen Genom durch intermolekulare Rekombination zwischen
(i) einem ersten DNA-Fragment, welches das gesamte oder einen Teil des Genoms des Stammvirus umfasst, und (ii) einem zweiten DNA-Fragment, welches eine exogene DNA-Sequenz, die von den zu (i) homologen, flankierenden Sequenzen A und B eingerahmt wird, umfasst, eine exogene DNA-Sequenz eingefügt wird, **dadurch gekennzeichnet, dass** die prokaryotische Zelle von einem *Escherichia coli* recBC sbcBC-Stamm abgeleitet ist.

2. Verfahren gemäß Anspruch 1, **dadurch gekennzeichnet, dass** es sich bei dem Stammvirus um ein Adenovirus, Retrovirus, ein mit dem Adenovirus verwandtes Virus, ein Pockenvirus oder ein Herpesvirus handelt.

3. Verfahren gemäß Anspruch 2, **dadurch gekennzeichnet, dass** es sich bei dem Stammvirus um ein menschliches, ein Hunde-, Vogel-, Rinder-, Maus-, Schafs-, Schweine- oder Affenadenovirus oder ein hybrides Adenovirus handelt.

4. Verfahren gemäß Anspruch 3, **dadurch gekennzeichnet, dass** das Stammvirus ein Hundevirus des CAV-2-Typs ist.

5. Verfahren gemäß Anspruch 3, **dadurch gekennzeichnet, dass** das Stammvirus ein humanes Adenovirus des Serotyps C ist.

6. Verfahren gemäß Anspruch 5, **dadurch gekennzeichnet, dass** das Stammvirus ein humanes Adenovirus des Typs 5 ist.

7. Verfahren nach einem der Ansprüche 1 bis 6, **dadurch gekennzeichnet, dass** die exogene DNA-Sequenz für ein Polypeptid von therapeutischem Interesse aus der Gruppe der Koagulationsfaktoren, Wachstumshormone, Zytokine, Lymphokine, tumorsuppressiven Polypeptide, zellulären Rezeptoren, zellulären Rezeptorliganden, Proteaseinhibitoren, Antikörper, Toxine, Immunotoxine, Dystrophine oder Polypeptide, die in die zellulären Ionenkanäle eingreifen, wie das CFTR-Protein, kodiert.

8. Verfahren nach einem der Ansprüche 1 bis 7, **dadurch gekennzeichnet, dass** die homologen, flankierenden Sequenzen A und B eine Länge von 10 bp bis 10 kb, vorteilhafterweise von 20 bp bis 5 kb, bevorzugt von 30 bp bis 2 kb, und besonders bevorzugt von 40 bp bis 1 kb, aufweisen.

9. Verfahren nach einem der Ansprüche 1 bis 8, **dadurch gekennzeichnet, dass** das erste DNA-Fragment auf Höhe des Insertionsbereichs der exogenen Sequenz linearisiert ist.

10. Verfahren nach einem der Ansprüche 1 bis 9 zur Herstellung eines rekombinanten viralen Vektors, der für die Replikation defektiv ist.

11. Verfahren gemäß Anspruch 10 zur Herstellung eines rekombinanten adenoviralen Vektors, dem die gesamte oder ein Teil wenigstens eines für die Replikation essentiellen Bereichs E1, E2 und/oder E4 fehlt.

12. Verfahren gemäß Anspruch 11, **dadurch gekennzeichnet, dass** dem rekombinanten adenoviralen Vektor außerdem ein Teil oder der gesamte Bereich E3 fehlt.

13. Verfahren nach einem der Ansprüche 1 bis 12 zur Herstellung eines rekombinanten viralen Vektors von wenigstens 30 kb.

14. Verfahren nach den Ansprüchen 1 bis 13 durch intermolekulare Rekombination zwischen (i) einem ersten DNA-Fragment, welches das gesamte oder einen Teil des Genoms des Stammvirus umfasst, (ii) einem zweiten DNA-Fragment, welches einen ersten Teil der DNA-Sequenz von Interesse, welche an ihrem 5'-Ende mit der flankierenden Sequenz A versehen ist, enthält, und (iii) einem dritten DNA-Fragment, welches einen zweiten Teil der DNA-Sequenz von Interesse, welche an ihrem 3'-Ende mit der flankierenden Sequenz B versehen ist, enthält, wobei die zweiten und dritten DNA-Fragmente an ihren jeweiligen 3'- und 5'-Enden eine homologe Sequenz aufweisen.

15. Verfahren nach einem der Ansprüche 1 bis 14 zum Einführen einer Modifikation durch Deletion, Mutation und/oder Substitution eines oder mehrerer Nukleotide oder einer exogenen DNA-Sequenz in ein virales Genom.

16. Verwendung eines *E. coli* recBC sbcBC-Stammes zur Klonierung von DNA-Fragmenten in einen Vektor gemäß Anspruch 1 durch intermolekulare homologe Rekombination.

## Claims

1. Method for preparing, in a prokaryotic cell, a recombinant viral vector of at least 20 kb derived from a parent virus into the genome of which an exogenous DNA sequence is inserted, by intermolecular recombination between (i) a first DNA fragment comprising all or part of said genome of the parent virus and (ii) a second DNA fragment comprising said exogenous DNA sequence surrounded by flanking sequences A and B which are homologous to (i), **characterized in that** said prokaryotic cell is derived from a recBC sbcBC strain of *Escherichia coli*.

2. Method according to Claim 1, **characterized in that** the parent virus is selected from the group consisting of adenoviruses, retroviruses, adeno-associated viruses, poxviruses and herpesviruses.

3. Method according to Claim 2, **characterized in that** the parent virus is an adenovirus of human, canine, avian, bovine, murine, ovine, porcine or simian origin, or alternatively a hybrid adenovirus.

4. Method according to Claim 3, **characterized in that** the parent virus is a type CAV-2 adenovirus of canine origin.

5. Method according to Claim 3, **characterized in that** the parent virus is a serotype C adenovirus of human origin.

6. Method according to Claim 5, **characterized in that** the parent virus is a type 5 adenovirus of human origin.

7. Method according to one of Claims 1 to 6, **characterized in that** said exogenous DNA sequence codes for a polypeptide of therapeutic interest selected from the group consisting of coagulation factors, growth hormones, cytokines, lymphokines, tumour-suppressing polypeptides, cell receptors, ligands for cell receptors, protease inhibitors, antibodies, toxins, immunotoxins, dystrophin and polypeptides participating in cellular ion channels, such as CFTR protein.

8. Method according to one of Claims 1 to 7, **characterized in that** the homologous flanking sequences A and B are from 10 bp to 10 kb, advantageously from 20 bp to 5 kb, preferably from 30 bp to 2 kb, and as an absolute preference from 40 bp to 1 kb, in length.

9. Method according to one of Claims 1 to 8, **characterized in that** the first DNA fragment is linearized in the insertion region of the exogenous sequence.

10. Method according to one of Claims 1 to 9, for the preparation of a recombinant viral vector which is defective for replication.

11. Method according to Claim 10, for the preparation of a recombinant adenoviral vector lacking all or part of at least one region essential for replication, selected from the E1, E2 and E4 regions.

12. Method according to Claim 11, **characterized in that** the recombinant adenoviral vector lacks, in addition, all or part of the E3 region.

13. Method according to one of Claims 1 to 12, for the preparation of a recombinant viral vector of at least 30 kb.

14. Method according to Claims 1 to 13, by intermolecular recombination between (i) a first DNA fragment comprising all or part of said genome of the parent virus, (ii) a second DNA fragment comprising a first portion of said DNA sequence of interest equipped at its 5' end with said flanking sequence A and (iii) a third DNA fragment comprising a second portion of said DNA sequence of interest equipped at its 3' end with said flanking sequence B; said second and third DNA fragments containing a homologous sequence at their respective 3' and 5' ends.

15. Method according to one of Claims 1 to 14, for introducing a modification by deletion, mutation and/or substitution of one or more nucleotides or an exogenous DNA sequence into a viral genome.

16. Use of a recBC sbcBC strain of *E. coli* for the cloning of DNA fragments into a vector according to Claim 1 by intermolecular homologous recombination.


### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 22 58 07
   (ii) TITRE DE L' INVENTION: Nouveau procède de préparation d'un vecteur viral
   (iii) NOMBRE DE SEQUENCES: 10
   (iv) FORME LISIBLE PAR ORDINATEUR:
      (A) TYPE DE SUPPORT: Tape
      (B) ORDINATEUR: I3M PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS : NON
   (vi) ORIGINE :
      (A) ORGANISME: adenovirus humain
      (B) SOUCHE: serotype 5
      (C) INDIVIDUEL ISOLE: oligonucleotide de synthèse OTG6597
   (xi) DESCRIPTION DE LA SEQUENCE: SEQ ID NO: 1:
   GCCGAATTCT TAATTAACAT CATCAATAAT ATACCTTA 38
(2) INFORMATION POUR LA SEQ ID NO: 2:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: adenovirus humain
      (B) SOUCHE: serotype 5
      (C) INDIVIDUEL ISOLE: oligonucleotide de synthese OTG6598
   (xi) DESCRIPTION DE LA SEQUENCE: SEQ ID NO: 2:
   GACAGATCTG TCGACGTGGC AGGTAAGATC GATCA 35
(2) INFORMATION POUR LA SEQ ID NO: 3:
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: aàenovirus humain
      (B) SOUCHE: serotype 5
      (C) INDIVIDUEL ISOLE: oligonucleotide de synthese OTG6599
   (xi) DESCRIPTION DE LA SEQUENCE: SEQ ID NO: 3:
   AGGAGATCTG TCGACTCTCA AACATGTCTG CGGGT 35
(2) INFORMATION POUR LA SEQ ID NO: 4:
   (i) CARACTERISTIQUES DE LA SEQUENCE:
      (A) LONGUEUR: 47 paires de bases
      (B) TYPE: acide nucléique
      (C) NOMBRE DE BRINS: simple
      (D) CONFIGURATION. linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Rous sarcoma virus
      (C) INDIVIDUEL ISOLE: oligonucleotide de synthese OTG5892
   (xi) DESCRIPTION DE LA SEQUENCE: SEQ ID NO: 4:
   GTCGTAGGAT CCAGCTGCTC CCTGCTTGTG TGTTGGAGGT CGCTGAG 47
(2) INFORMATION POUR LA SEQ ID NO: 5:
   (i) CARACTERISTIQUES DE LA SEQUENCE:
      (A) LONGUEUR: 47 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Rous sarcoma virus
      (C) INDIVIDUEL ISOLE: oligonucleotide de synthese OTG5893
   (xi) DESCRIPTION DE LA SEQUENCE: SEQ ID NO: 5:
   GTAGCTGACG TCCCAGGTGC ACACCAATGT GGTGAATGGT CAAATGG 47
(2) INFORMATION POUR LA SEQ ID NO: 6:
   (i) CARACTERISTIQUES DE LA SEQUENCE:
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: adenovirus humain
      (B) SOUCHE: serotype 5
      (C) INDIVIDUEL ISOLE: oligonucleotiàe de synthese OTG5455
   (xi) DESCRIPTION DE LA SEQUENCE: SEQ ID NO: 6:
   ATCGGAATTC AAGATGATTA GGTAC 25
(2) INFORMATION POUR LA SEQ ID NO: 7:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: adenovirus humain
      (B) SOUCHE: serotype 5
      (C) INDIVIDUEL ISOLE: oligonucleotide de synthèse OTG5456
   (xi) DESCRIPTION DE LA SEQUENCE: SEQ ID NO: 7:
   ATCGTCTAGA TTAAGGCATT TTCTTTTC 28
(2) INFORMATION POUR LA SEQ ID NO: 8:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: adenovirus canin
      (B) SOUCHE: CAV-2
      (C) INDIVIDUEL ISOLE: oligonucleotide de synthèse OTG6974
   (xi) DESCRIPTION DE LA SEQUENCE: SEQ ID NO: S:
   CAGGGATCCG CGGCCGCATC ATCAATAATA TACAGG 36
(2) INFORMATION POUR LA SEQ ID NO: 9:
   (i) CARACTERISTIQUES DE LA SEQUENCE:
      (A) LONGUEUR: 29 paires de bases
      (B) TYPE: acide nucléique
      (C) NOMBRE DE BRINS : simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: adenovirus canin
      (B) SOUCHE: CAV-2
      (C) INDIVIDUEL ISOLE: oligonucleotide de synthese OTG6975
   (xi) DESCRIPTION DE LA SEQUENCE: SEQ ID NO: 9:
   CTGCTGCAGT CAGAAATGCT AGCAGGAGA 29
(2) INFORMATION POUR LA SEQ ID NO: 10:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: adenovirus canin
      (B) SOUCHE: CAV-2
      (C) INDIVIDUEL ISOLE: oligonucleotide de synthese OTG6976
   (xi) DESCRIPTION DE LA SEQUENCE: SEQ ID NO: 10:
   TGCGGATCCA CAGACTAAGC GGAGGTA                                      27